(19) Europäisches Patentamt
European Patent Office
Office européen des brevets

(11) Publication number: 0 012 510 B1

(12) **EUROPEAN PATENT SPECIFICATION**

(45) Date of publication of patent specification: 08.09.82

(51) Int. Cl.[3]: C 07 D 251/32, C 07 D 251/36

(21) Application number: 79302504.0

(22) Date of filing: 08.11.79

(54) **Process for treating an effluent containing cyanuric acid values.**

(30) Priority: 06.12.78 GB 4742478
06.12.78 GB 4742578
06.12.78 GB 4742678

(43) Date of publication of application:
25.06.80 Bulletin 80/13

(45) Publication of the grant of the patent:
08.09.82 Bulletin 82/36

(84) Designated Contracting States:
AT BE CH DE FR GB IT LU NL SE

(56) References cited:
GB - A - 1 487 707
US - A - 3 846 424

(73) Proprietor: CHLOR-CHEM LIMITED
Hauxton, Cambridge CB2 5HU (GB)

(72) Inventor: Haji, Faraj Hassan
5 Hawthorn Avenue
Hauxton Cambridge CB2 5JA (GB)
Inventor: Harris, John Frederick
13 North End Meldreth
Nr. Royston Hertfordshire (GB)
Inventor: Shuttlewood, Victor Cyril
53 Hallfields Road
Tarvin Chester (GB)

(74) Representative: Richer, David Leonard Industrial Property Department FBC Limited
Chesterford Park Research Station
Saffron Walden, Essex CB10 1XL (GB)

EP 0 012 510 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for treating an effluent containing cyanuric acid values

The present invention relates to a process for treating an effluent containing cyanuric acid values notably one from a cyanuric acid chlorination process.

The production of cyanuric acid and/or chlorinated cyanuric acids gives rise to effluents which contain levels of nitrogen and cyanuric acid values which are too high to permit direct discharge of the effluents to waste. Furthermore, the nitrogen and cyanuric acid values are valuable components which represent a cash loss when simply discharged to waste. It has been proposed to treat these effluents to render them suitable for discharge to waste or to recover some of the nitrogen values for use as fertilizers. For example, in UK Patent No. 1487707 and US Patent No. 3846424 there are described processes in which chloroisocyanurates are decomposed by strong acids to form cyanuric acid which are then precipitated by treatment with an alkali. However, such proposals have either not recovered the cyanuric acid values or have required the use of expensive treatment processes and chemicals. To date no simple and effective method exists for treating these effluents.

We have not devised a simple process for treating an effluent containing cyanuric acid values. The term cyanuric acid values is used herein to denote collectively or individually cyanuric acid, isocyanuric acid, salts of these acids, halogenated derivatives of these acids and/or salts of these derivatives.

Accordingly, the present invention provides a process which comprises treating a first reagent containing a halogen donor with a second reagent containing $NH_3$ and/or $NH_4^+$ ions, at least one of the reagents also providing cyanuric acid values, whereby there is formed a reaction mixture containing a haloamine; decomposing the haloamine to give off nitrogen whereby the nitrogen content of the reaction mixture is reduced; and recovering cyanuric acid or a salt thereof from the resultant mixture.

The reagents for present use can be derived from a wide range of sources and at least one of the reagents is preferably an effluent from a process which uses or produces cyanuric acid, a halogenated cyanuric acid or a salt thereof. The invention is of especial use in treating the aqueous effluent from a cyanuric acid chlorination process. For convenience the invention will be described hereinafter with respect to this especially preferred use.

The effluent from a cyanuric acid chlorination process is typically an aqueous medium containing 0.1 to 1.0% w/w chlorine, mainly as chlorinated cyanuric acids, and 5 to 10% w/w sodium chloride, and will usually be acidic.

The chlorinated cyanuric acid in the aqueous medium includes those of the general formula:

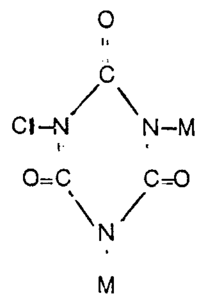

and hydrates thereof or the enol forms thereof wherein M is selected from halogen, notably chlorine, hydrogen or an alkali-metal. It is preferred that at least one M be chlorine. Suitable chlorinated cyanuric acids thus include di- or trichloroisocyanuric acids, monosodium dichloroisocyanurate, monopotassium dichloroisocyanurate; and hydrates thereof. Mixtures of chlorinated cyanuric acids can be present in the aqueous medium.

It is preferred that the effluent containing the chlorinated cyanuric acids has a high enough pH value, typically above 5, preferably 5 to 9, to reduce the rate of decomposition of the chlorinated cyanuric acid to an acceptable level, notably where the effluent is to be stored before use. The desired pH value is conveniently achieved by the addition of a base, e.g. an alkali-metal or alkaline-earth metal hydroxide, carbonate or bicarbonate to the effluent.

In the process of the invention sufficient $NH_3$ and/or $NH_4^+$ is added to the effluent to convert at least part of the chlorinated cyanuric acids therein into chloramine and cyanuric acid. We believe that the chlorinated cyanuric acids react by forming $OCl^-$ and cyanuric acid and that the $OCl^-$ then reacts with the $NH_3$ or $NH_4^+$ to form chloramines. The chloramines are unstable and decompose to give off nitrogen. The stoichiometric amount of $NH_3$ and $NH_4^+$ required to react with the chlorinated cyanuric acids is 2/3 of a mol of $NH_3$ or $NH_4^+$ per mol of $OCl^-$ from the chlorinated cyanuric acid. However, an excess or deficiency can be used, depending upon the degree of conversion required and the cost of the reagents. Typically, the chlorinated cyanuric acid will be used in such amount as to provide from 3.0 to 0.75 mols of $OCl^-$ per molar proportion of $NH_4^+$ or $NH_3$. If desired, the chlorinated cyanuric acid can be used in admixture with other halogen donors, e.g. inorganic hypochlorites, notably when an effluent stream is used to provide the $NH_3$ or $NH_4^+$ and is in excess of that required to react with the $OCl^-$ provided by the chlorinated cyanuric acid alone. Whilst the desired proportions of $NH_3$ or $NH_4^+$ to $OCl^-$ can be achieved by monitoring the flow rate of reagents to a reaction vessel, this can be difficult, notably where the composition of the reagents varies. We have found that monitoring the redox potential of the reaction mixture provides a convenient method for controlling the rate of addition of one or both reagents. We prefer to maintain the redox potential of the reaction mixture at from 500 to 700 millivolts and to control the rate of addition of the $NH_3$ or $NH_4^+$ in response to the signal from a redox potential electrode.

The $NH_3$ or $NH_4^+$ can be provided by the use of ammonia, e.g. as the aqueous ammonia by-product obtained by scrubbing the off-gases from the urea calcination stage in the production of cyanuric acid. Alternatively, the $NH_4^+$ can be provided in the form of urea and/or an ammonium salt of a mineral acid, e.g. of sulphuric, nitric, hydrochloric and/or phosphoric acid. A preferred source of $NH_4^+$ ions for present use is the aqueous effluent from a cyanuric acid production process. This effluent is typically an aqueous solution containing ammonium salts of hydrochloric, sulphuric, nitric and/or phosphoric acids as well as some cyanuric acid. An alternative source of $NH_4^+$ is the effluent from an azodicarbonamide production process and/or the water spillages collected from a cyanuric acid production process which typically contain $NH_4^+$ and urea. If desired, both gaseous $NH_3$ and an aqueous solution containing $NH_4^+$ can be reacted with the chlorinated cyanuric acid effluent. The $NH_3$ or $NH_4^+$ source can be added as a continuous or semicontinuous operation. Preferably, the reaction mixture is agitated during the addition of the $NH_3$ or $NH_4^+$ source to the aqueous chlorinted cyanuric acid effluent or vice versa.

The reaction can be carried out at any suitable temperature, e.g up to the boiling point of the reaction mixture, but preferably in the range ambient (e.g. 10°C) up to 75°C.

It is preferred to carry out the reaction between the chlorinated cyanuric acid effluent and the $NH_3$ or $NH_4^+$ at a pH in the range 7 to 11, so as to reduce the formation of nitrogen trichloride in the reaction mixture. It will usually be necessary to add a base to the reaction mixture and/or to the reagents to achieve these pH levels. Suitable bases include alkali-metal and/or alkaline-earth metal hydroxides, carbonates and/or bicarbonates. To aid recovery of the cyanuric acid values from the reaction mixture the base is preferably one which forms an insoluble salt of cyanuric acid, e.g. sodium, potassium, barium, magnesium or calcium hydroxide. It is also preferred to add sufficient base to form the mono- salt of the cyanuric acid present or formed in the reaction mixtures. This will usually be achieved by operating with a pH value within the range 8 to 10. Preferably, the base is added in the form of an aqueous solution or suspension thereof. Commercially available purity bases can be used.

The decomposition of the chloramines formed in the reaction mixture from the $NH_3$ or $NH_4^+$ and the chlorinated cyanuric acid will usually occur spontaneously. However, it may be desired to assist evolution of nitrogen, e.g. by stirring or agitating the reaction mixture, and to hold the reaction mixture for a time, e.g. 0.1 to 3 hours, to permit the decomposition to occur.

Where an excess of $OCl^-$ over the stoichiometric is used so as to reduce the nitrogen content of the final effluent to a minimum, it may be desired to destroy the excess $OCl^-$ in the reaction mixture, e.g. by treatment of the reaction mixture with a sulphite, thiosulphite or with $SO_2$.

The cyanuric acid or salt thereof can be recovered from the reaction mixture in a number of ways. Thus, the reaction mixture can be cooled to precipitate cyanuric acid or a salt thereof. Where a base giving an insoluble or slightly soluble salt with cyanuric acid is used, the precipitated salt can be recovered from the reaction mixture using conventional techniques, e.g. filtration, centrifuging or decantation. If desired, the reaction mixture can be cooled, e.g. to from 0 to 5°C, to cause further precipitation of the cyanuric acid salt prior to, during or after the initial separation described above. Several precipitation and separation stages can be used.

The resultant aqueous effluent contains a lower nitrogen and cyanuric acid value content than initially, and has a higher pH and is more suitable for discharge to waste. Where the gaseous and/or liquid effluent from a cyanuric acid production plant is used to provide the $NH_3$ or $NH_4^+$, the process of the invention offers a particularly preferred method for treating both effluents simultaneously to recover cyanuric acid values therefrom and reduce the nitrogen content thereof. The recovered cyanuric acid values are conveniently recycled to a chlorination plant for conversion to chlorinated cyanuric acids. For such re-use the recovered acid is usually dissolved in an aqueous alkali and it is preferred to filter this solution before chlorination to remove insoluble impurities, e.g. iron.

Whilst the invention has been described above with respect to the treatment of an effluent containing a chlorinated cyanuric acid, it is within the scope of the present invention to treat an effluent containing a brominated, or other halogenated cyanuric acid. The term chlorinated cyanuric acid is therefore to be construed herein as including such other halogenated cyanuric acids.

As indicated earlier, the invention has been described with respect to the treatment of an effluent from the production of a chlorinated cyanuric acid. However, the invention can be applied also to other effluents containing chlorinated cyanuric acids, e.g. the effluents from textile bleaching or scouring processes. Also the invention can be applied to the treatment of an effluent containing $NH_4^+$ and preferably also cyanuric acid values. In this case the treatment with the halogen donor is used to reduce the nitrogen content of the effluent. The halogen donor can be a chlorinated cyanuric

acid (e.g. derived from the effluent from a cyanuric acid chlorination process) or can be a hypohalite (e.g. calcium or sodium hypochlorite), an organic compound (e.g. an halogenated alkyl hydantoin or a sulphonchloramide) or gaseous chlorine or bromine. The reaction between the halogen donor and the effluent containing $NH_4^+$ and cyanuric acid values and the recovery of the cyanuric acid is carried out as described above. The effluent to be treated with the halogen donor can be derived from a number of sources, but is preferably the aqueous effluent from a cyanuric acid production process. This typically is an aqueous solution or suspension containing cyanuric acid or a salt thereof (generally 0.5 to 1.5% w/w), ammonium sulphates, phosphates, nitrate and/or chloride (generally 0.5 to 1.5% w/w $NH_4^+$) and will usually be strongly acidic. As described above with respect to the treatment of the chlorinated cyanuric acid effluent, the cyanuric acid effluent is preferably treated with a base to raise the pH value thereof to a value within the range 8—10 before or during treatment with the halogen donor.

The present invention will now be illustrated by the following Examples in which all parts and percentages are by weight unless stated otherwise:

Example 1

The aqueous effluent from a cyanuric acid plant (200 parts, 0.8% cyanuric acid, 0.79% $NH_3$ equivalent and 18% $H_2SO_4$ equivalent) was treated in a stirred tank reactor with IO N sodium hydroxide solution (82.3 parts) to give a pH value in the effluent of 9.0. The aqueous effluent from a cyanuric acid chlorination plant (1080.5 parts containing chlorinated cyanuric acids providing the equivalent of 0.62% chlorine as $OCl^-$ and whose pH had been adjusted by the addition of sodium hydroxide to a value of 6.2 to 6.3) was added to the reactor. The pH was maintained at 9.0 by the addition of further sodium hydroxide and the temperature of the resultant mixture was 34°C.

Further quantities of both effluents were added in the ratios specified above with removal of a similar volume of reaction mixture. The reaction mixture was maintained at pH 9 and 34°C and the average residence time of reagents in the reactor was approximately $1\frac{1}{4}$ hours.

The removed reaction mixture was filtered to give monosodium cyanurate crystals at approximately 85% recovery of the cyanuric acid values in the feed materials. The aqueous filtrate contained only 0.004% $NCl_3$ 0.19% cyanuric acid as a sodium salt and 0.035% $NH_4^+$, and was more suitable for direct discharge to waste.

Example 2

The aqueous effluent from a cyanuric acid chlorination plant (5570 parts by volume containing chlorinated cyanuric acids providing the equivalent of 513.8 parts of chlorine as $OCl^-$ and whose pH had been adjusted by the addition of sodium hydroxide to a value of 6.5 to 6.8) was fed to a stirred tank reactor equipped with a pH probe and a redox electrode. Aqueous ammonia (10.17% w/v) as the scrubbing liquors from the off-gases of a urea calcination plant was fed to the stirred reactor in response to the signal from the redox electrode. The rate of feed of the aqueous effluent was substantially constant and the ammonia feed was adjusted to maintain a redox potential of −550 to −600 mV in the reaction mixture. Sodium hydroxide was added to maintain the pH at between 8.6 and 9.4. The temperature of the reaction mixture was 18°C and the residence time of the reagents in the reaction vessel was $1\frac{1}{2}$ hours.

Reaction mixture was removed to maintain the level in the reaction vessel and filtered to recover the monosodium cyanurate crystals which had formed. Approximately 97% of the cyanuric acid values in the feed materials were recovered. These were dissolved in sodium hydroxide solution and the solution filtered to remove insoluble matter, e.g. iron hydroxide. The solution was suitable for recycling to the chlorination process.

The filtrate from the reaction mixture contained 0.04% sodium cyanurate, 0.016% $NH_4^+$ and was suitable for direct discharge to waste.

**Claims**

1. A process for treating an aqueous medium containing cyanuric acid values consisting of one or more of cyanuric acid, isocyanuric acid, salts thereof, and halogenated derivatives of said acids and salts, characterised in that a first rêagent containing a halogen donor is treated with a second reagent providing $NH_3$ and/or $NH_4^+$ ions, at least one of the reagents being an aqueous medium containing the cyanuric acid values, whereby there is formed a reaction mixture containing a haloamine; decomposing the haloamine to give off nitrogen whereby the nitrogen content of the reaction mixture is reduced; and recovering cyanuric acid or a salt thereof from the resultant mixture.

2. A process as claimed in claim 1 characterised in that the first reagent is an aqueous medium containing an halogenated cyanuric acid.

3. A process as claimed in claim 1 or claim 2 characterised in that the second reagent comprises a by-product or effluent from a cyanuric acid plant.

4. A process as claimed in claim 3 characterised in that said second reagent comprises aqueous ammonia obtained by scrubbing the off-gases from the calcination of urea.

5. A process according to claim 1 characterised in that an aqueous effluent containing an halogenated cyanuric acid or a salt thereof is reacted with an aqueous effluent

containing $NH_3$ and/or $NH_4^+$ and optionally also cyanuric acid or a salt thereof whereby there is formed a reaction mixture containing a haloamine; decomposing the haloamine to give off nitrogen whereby the nitrogen content of the reaction mixtures is reduced; and recovering cyanuric acid or a salt thereof from the resultant mixture.

6. A process according to any one of the preceding claims characterised in that the first reagent provides from 0.75 to 3.0 mols of $OCl^-$ per mol of $NH_3$ or $NH_4^+$ provided by the second reagent.

7. A process according to any one of the preceding claims characterised in that the reaction between the first and second reagent is carried out at a pH of from 7 to 11 and at a redox potential of from 500 to 700 millivolts.

**Revendications**

1. Procédé pour traiter un milieu aqueux contenant des produits à base d'acide cyanurique consistant en un ou plusieurs produits choisis parmi l'acide cyanurique, l'acide isocyanurique, leurs sels et des dérivés halogénés desdits acides et sels, caractérisé en ce qu'on traite un premier corps destiné à réagir, contenant un donneur d'halogène, avec un second corps destiné à réagir et fournissant $NH_3$ et/ou des ions $NH_4^+$, au moins l'un des corps destinés à réagir étant un milieu aqueux contenant les produits à base d'acide cyanurique, de manière à former un mélange réactionnel contenant une halogénoamine; on décompose l'halogénoamine pour dégager l'azote de manière à diminuer la teneur en azote du mélange réactionnel; et l'on recueille du mélange résultant l'acide cyanurique ou un de ses sels.

2. Procédé selon la revendication 1, caractérisé en ce que le premier corps destiné à réagir est un milieu aqueux contenant un acide cyanurique halogéné.

3. Procédé selon la revendication 1 ou la revendication 2, caractérisé en ce que le second corps destiné à réagir comprend un sous-produit ou effluent provenant d'une installation de production d'acide cyanurique.

4. Procédé selon la revendication 3, caractérisé en ce que le second corps destiné à réagir comprend de l'ammoniaque aqueuse obtenue par lavage des gaz dégagés lors de la calcination de l'urée.

5. Procédé selon la revendication 1, caractérisé en ce qu'on fait réagir un effluent aqueux, contenant un acide cyanurique halogéné ou un ses sels, avec un effluent aqueux contenant $NH_3$ et/ou $NH_4^+$ et éventuellement aussi de l'acide cyanurique ou un de ses sels, de manière à former un mélange réactionnel contenant une halogénoamine; on décompose l'halogénoamine pour dégager l'azote de manière à diminuer la teneur en azote des mélanges réactionnels; et l'on récupère l'acide cyanurique ou un de ses sels à partir du mélange résultant.

6. Procédé selon l'une quelconque des revendications précédentes, caractérisé en ce que le premier corps destiné a réagir fournit de 0,75 à 3,0 moles de $OCl^-$ par mole de $NH_3$ ou de $NH_4^+$ fourni par le second corps destiné à réagir.

7. Procédé selon l'une quelconque des revendications précédentes, caractérisé en ce qu'on effectue la réaction entre le premier et le second corps destinés à réagir à un pH de 7 à 11 et à un potentiel d'oxydo-réduction de 500 à 700 mV.

**Patentansprüche**

1. Verfahren zum Behandeln eines wässerigen Mediums, das Cyanursäurewerte bestehend aus Cyanursäure, Isocyanursäure, Salzen hievon und/oder halogenierten Derivaten der genannten Säuren und Salze enthält, dadurch gekennzeichnet, daß ein erstes Reagens, das einen Halogendonator enthält, mit einem zweiten Reagens, das $NH_3$ und/oder $NH_4^+$-Ionen liefert, wobei zumindest eines der Reagentien ein wässeriges Medium ist, das die Cyanursäurewerte enthält, behandelt wird, wodurch eine Reaktionsmischung gebildet wird, die ein Halogenamin enthält; das Halogenamin zersetzt wird, um Stickstoff abzugeben, wodurch der Stickstoffgehalt der Reaktionsmischung vermindert wird; und Cyanursäure oder ein Salz hievon aus der resultierenden Mischung gewonnen wird.

2. Verfahren nach Anspruch 1, dadurch gekennzeichnet, daß das erste Reagens ein wässeriges Medium ist, das eine halogenierte Cyanursäure enthält.

3. Verfahren nach Anspruch 1 oder 2, dadurch gekennzeichnet, daß das zweite Reagens ein Nebenprodukt oder einen Abfluß einer Cyanursäureanlage umfaßt.

4. Verfahren nach Anspruch 3, dadurch gekennzeichnet, daß das genannte zweite Reagens wässeriges Ammoniak umfaßt, das durch Waschen der Abgase von der Kalzinierung von Harnstoff erhalten wurde.

5. Verfahren nach Anspruch 1, dadurch gekennzeichnet, daß ein wässeriger Abfluß, der eine halogenierte Cyanursäure oder ein Salz hievon enthält, mit einem wässerigen Abfluß, der $NH_3$ und/oder $NH_4^+$ und gegebenenfalls auch Cyanursäure oder ein Salz hievon enthält, umgesetzt wird, wodurch eine Reaktionsmischung gebildet wird, die ein Halogenamin enthält; das Halogenamin zersetzt wird, um Stickstoff abzugeben, wodurch der Stickstoffgehalt der Reaktionsmischungen vermindert wird; und Cyanursäure oder ein Salz hievon aus der resultierenden Mischung gewonnen wird.

6. Verfahren nach einem der vorhergehenden Ansprüche, dadurch gekennzeichnet, daß das erste Reagens 0,75 bis 3,0 Mol $OCl^-$ pro Mol $NH_3$ oder $NH_4^+$, die durch das zweite Reagens geliefert werden, liefert.

7. Verfahren nach einem der vorhergehenden

Ansprüche, dadurch gekennzeichnet, daß die Reaktion zwischen dem ersten und dem zweiten Reagens bei einem pH von 7 bis 11 und bei einem Redox-Potential von 500 bis 700 mV durchgeführt wird.